# FASCICULE DE BREVET EUROPEEN

(11) **EP 1 385 572 B1**
(45) Date de publication et mention de la délivrance du brevet: **10.11.2010**
(21) Numéro de dépôt: 02738214.2
(22) Date de dépôt: 06.05.2002
(51) Int. Cl.: A61N 1/32

(54) **APPAREIL DE STIMULATION ET DE TRAITEMENT DE LA PEAU**
VORRICHTUNG ZUR STIMULATION UND BEHANDLUNG DER HAUT
APPARATUS FOR SKIN STIMULATION AND TREATMENT

(30) Priorité: 10.05.2001 FR 0106184; 11.09.2001 FR 0111729
(43) Date de publication de la demande: 04.02.2004
(73) Titulaire: Simonin, Philippe, 75017 Paris (FR)
(72) Inventeur: Simonin, Philippe, 75017 Paris (FR)
(74) Mandataire: Quantin, Bruno Marie Henri
(86) Numéro de dépôt international: PCT/FR2002/001547
(87) Numéro de publication internationale: WO 2002/089910

(56) Documents cités:
- EP-A- 0 387 176
- EP-A- 0 592 851
- FR-A- 2 418 646
- FR-A- 2 584 611
- FR-A- 2 665 366
- FR-A- 2 710 542
- FR-A- 2 753 385
- US-A- 4 856 526

## Description

L'invention concerne un appareil de stimulation et de traitement de la peau formant générateur d'impulsions électriques permettant d'appliquer lesdites impulsions, au moyen d'une électrode de forme adaptée, à la surface de la peau, pour des traitements localisés.

L'invention concerne notamment la nature du signal électrique délivré par ledit générateur d'impulsions. A titre d'application préférentielle, on a pu mettre en évidence que la mise en oeuvre de l'invention stimule de façon notable la repousse des implants capillaires.

On connaît du brevet français N° 2 753 385 un appareil électrique formant générateur d'impulsions pour, notamment, le traitement des rides.

Le document EP-A-0 592 851 décrit un dispositif de stimulation et traitement ayant une électrode connectée aux moyens generatéurs ayant une fréquence de l'ordre de grandeur de 1 MHz, modulée en amplitude par un signal basse fréquence ayant une fréquence de l'ordre de grandeur de 50 Hz.

L'invention concerne un nouveau type de générateur, de préférence associé à un nouveau type d'électrode, permettant des traitement particuliers notamment, l'accélération de la pousse des implants capillaires.

Plus précisément, l'invention concerne un appareil de stimulation et de traitement de la peau, comme défini dans la revendication 1.

Selon un mode de réalisation possible, ladite électrode d'application a la forme d'une fourche dont chaque branche est terminée par une petite excroissance, par exemple sensiblement sphérique. Selon une autre variante possible, l'électrode a la forme d'une fourche dont chaque branche est terminée par une petite barrette s'étendant transversalement.

Le signal haute fréquence a une fréquence de l'ordre de grandeur de 1 MHz tandis que le signal basse fréquence a une fréquence de l'ordre de grandeur de 50 Hz. Avantageusement, le signal basse fréquence peut être obtenu à partir de la tension alternative du réseau de distribution de l'électricité.

Pour combiner les deux signaux de fréquences différentes, on peut prévoir un hacheur piloté à ladite haute fréquence et recevant en entrée ledit signal basse fréquence. Le signal résultant peut être appliqué à l'électrode via un transformateur HF, dit transformateur d'impulsions, connecté entre le hacheur et ladite électrode. Ce transformateur d'impulsions, de faible puissance, permet d'élever la tension appliquée à l'électrode tout en limitant la valeur du courant à une valeur faible non dangereuse.

Un autre aspect concerne un instrument intégrant l'électrode et permettant au patient d'appliquer lui-même le traitement sans l'aide d'un praticien. En effet, de façon connue, lorsque le traitement doit être opéré par un praticien, l'appareil est muni d'une poignée métallique reliée par un fil électrique à l'une des bornes du générateur d'impulsions. L'autre borne du générateur est reliée par un autre fil électrique à un applicateur portant une électrode. Pendant le traitement, le patient tient la poignée à la main pendant que le praticien déplace l'électrode sur la zone de la peau à traiter. Un appareil de ce genre est peu pratique dès lors que le patient désire lui-même mettre en oeuvre le traitement, éventuellement à titre de complément, par exemple, à domicile. Un instrument particulier permet de surmonter cette difficulté.

Plus particulièrement, l'invention concerne aussi un instrument pour mise en oeuvre d'un traitement de la peau par électrothérapie, comportant un corps allongé formant élément de préhension, prolongé longitudinalement par une électrode reliée à un générateur électrique, **caractérisé en ce qu**'au moins une partie extérieure dudit corps est conductrice de l'électricité, électriquement isolée de ladite électrode et connectée à un premier fil électrique d'alimentation destiné à être relié audit générateur électrique.

Par exemple, la partie extérieure du corps peut comporter une enveloppe métallique et le traitement s'opère dès lors que le patient prend l'instrument en main en établissant le contact avec cette enveloppe métallique.

A titre d'exemple, l'intérieur du corps allongé précité peut être traversé par un second fil électrique d'alimentation connecté à ladite électrode et électriquement isolé de ladite partie extérieure dudit corps, conductrice de l'électricité. Avantageusement, lesdits premier et second fils électriques, isolés l'un de l'autre, font partie d'un cordon de liaison du type blindé. L'enveloppe métallique extérieure du blindage est reliée à la masse du générateur de signaux électriques auquel est relié l'instrument selon l'invention.

Cet instrument peut avantageusement constituer ou porter l'électrode du générateur décrit ci-dessus. Dans ce cas, par exemple, les deux fils électriques sont reliés à l'enroulement secondaire du transformateur d'impulsions. Cependant, ledit instrument peut être utilisé avec tout autre type d'appareil d'électrothérapie.

Avantageusement, une extrémité du corps est équipée d'un connecteur auquel ledit second fil électrique est électriquement connecté et ce connecteur est muni d'un embout de raccordement d'une électrode. L'électrode est donc amovible et on peut disposer d'un jeu d'électrodes de formes différentes, chaque électrode étant adaptable audit embout de raccordement. La forme de l'électrode est choisie en fonction de la nature du traitement et des résultats escomptés.

L'invention sera mieux comprise et d'autres avantages de celle-ci apparaîtront plus clairement à la lumière de la description qui va suivre d'un mode de réalisation possible d'un appareil conforme à son principe, donnée uniquement à titre d'exemple, et faite en référence aux dessins annexés dans lesquels :
- la figure 1 est un schéma bloc du générateur permettant d'élaborer le signal électrique désiré ;
- les figures 2 et 3 sont des exemples d'électrodes applicables notamment pour le traitement du cuir chevelu ;
- la figure 4 est un graphe illustrant la variation du signal délivré par le générateur de la figure 1 ;
- la figure 5 illustre un équipement complet pour la mise en oeuvre d'un traitement de la peau par électrothérapie ;
- la figure 6 est une vue en coupe et à plus grande échelle de l'instrument formant applicateur et muni d'une électrode amovible ; et
- la figure 7 illustre une autre forme d'électrode susceptible de remplacer celle de la figure 6, pour un autre type de traitement.

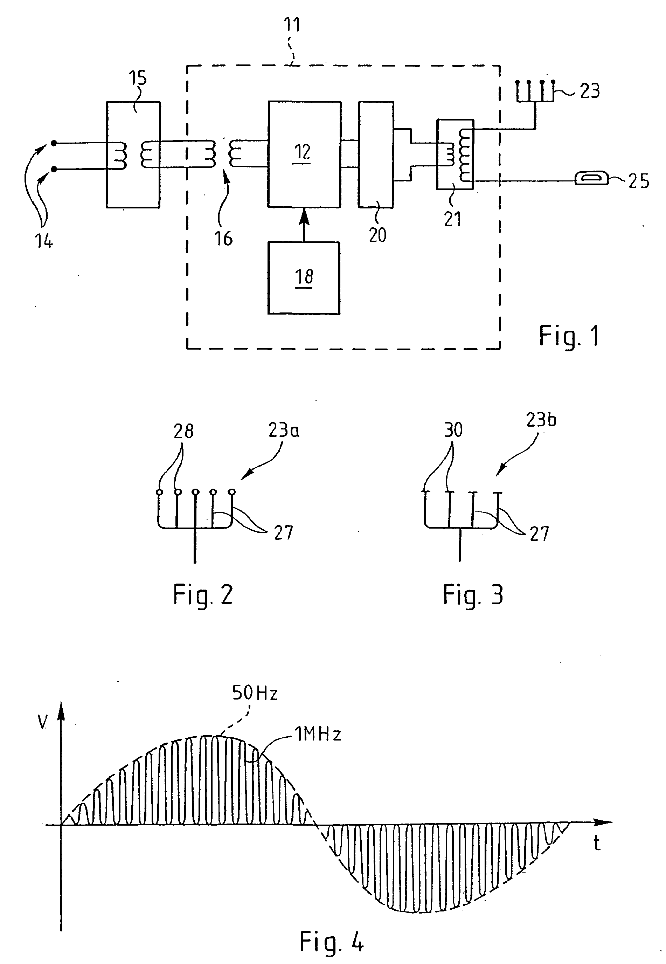

Sur la figure 1, on a représenté un générateur 11 comportant un hacheur 12 recevant en entrée une tension alternative à la fréquence de 50 Hz. Cette tension alternative peut être prélevée sur le réseau de distribution de courant, par exemple par l'intermédiaire d'un transformateur 15, abaisseur de tension situé à l'extérieur du boîtier du générateur 11. Le générateur 11 est relié au réseau de distribution de courant alternatif 14 (220V, 50 Hz) par le transformateur abaisseur de tension 15 délivrant une basse tension de 12V, 50 Hz. Le transformateur 15 alimente un transformateur élévateur 16, situé à l'intérieur du boîtier du générateur, qui restitue une tension de 220V environ, à l'entrée du hacheur 12. Par ailleurs, un oscillateur 18 de 1MHz est connecté à l'entrée de pilotage du hacheur. Il en résulte que le signal de sortie du hacheur est un signal haute fréquence (1 MHz) modulé en amplitude par le signal basse fréquence (50 Hz). Ce signal de sortie est appliqué à un circuit régulateur 20, limiteur de courant, dont la sortie est connectée à l'enroulement primaire d'un transformateur haute fréquence 21 : dit transformateur d'impulsions, élévateur de tension. L'une des bornes de l'enroulement secondaire est connecté à une électrode 23, métallique, tandis que l'autre borne est connectée à une prise de contact 25 ou analogue destinée à être mise en contact avec l'épiderme de la personne en cours de traitement. Cette prise peut être une poignée métallique tenue par cette personne pendant le traitement. L'électrode, quant à elle, sera déplacée lentement sur la zone à traiter de l'épiderme. Le signal V disponible à la sortie du hacheur est du type illustré à la figure 4. Les oscillations de haute fréquence ici à 1MHz évoluent en amplitude à l'intérieur d'une enveloppe de basse fréquence (50Hz). Bien entendu, les rapports des fréquences ne sont pas respectés sur la figure 4. Les alternances à 1 MHz s'inscrivent en amplitude dans une enveloppe à 50 Hz.

On peut penser que le signal haute fréquence, une fois élevé en tension et appliqué sur la peau, procure une stimulation des couches basales de l'épiderme tandis que la modulation de cette haute fréquence (l'enveloppe basse fréquence ) "traverse" la barrière épidermique, en stimulant les fibroblastes ce qui favorise la production de collagène et d'élastine. La haute fréquence, quant à elle, favorise la microcirculation artérielle veineuse et lymphatique.

Ce type de traitement, c'est à dire le fait d'appliquer ce signal sous une tension variable de 200 à 1200 V, via le transformateur d'impulsions, donne des résultats remarquables pour la diminution des rides superficielles et moyennes, améliore la tonicité cutanée et agit sur le métabolisme des cheveux : ralentissement de la chute, régulation de la fonction sébacée et sudoripare, augmentation du volume des cheveux, amélioration de la qualité des cheveux, repousse plus rapide des cheveux, notamment dans le cas d'implants.

Les figures 2 et 3 montrent deux types d'électrodes possibles. Ainsi, l'électrode 23a de la figure 2 a la forme générale d'une fourche comprenant plusieurs branches 27 parallèles (5 dans l'exemple représenté) et chaque branche est terminée par une petite excroissance 28, par exemple sensiblement sphérique. L'électrode 23b de la figure 3 a également la forme générale d'une fourche (à quatre branches dans l'exemple). Chaque branche 27 est terminée par une petite barrette 30 de faible largeur, s'étendant transversalement. Ces électrodes sont celles qui se sont révélées parmi les plus efficaces pour le traitement du cuir chevelu.

Selon les applications envisagées (traitement cutané ou traitement spécifique du cuir chevelu, notamment) les paramètres de fonctionnement seront adaptés, notamment la valeur de l'amplitude du signal.

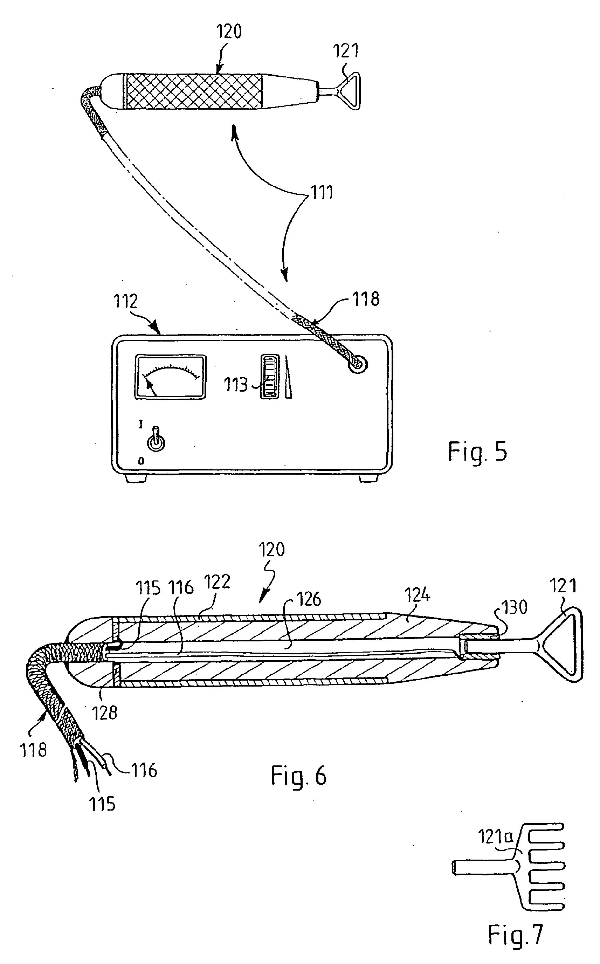

L'équipement 111 pour la mise en oeuvre du traitement de la peau par électrothérapie tel que représenté figure 5 se compose essentiellement d'un boîtier 112 renfermant un générateur électrique, par exemple un générateur d'impulsions, dont l'intensité peut être réglée au moyen d'un potentiomètre 113 accessible en façade. La sortie de ce générateur est reliée à deux fils électriques 115, 116 qui font partie d'un cordon de liaison 118 du type bifilaire blindé, lequel est raccordé à un instrument 120 pour la mise en oeuvre du traitement, cet instrument étant porteur d'une électrode 121. Le conducteur de blindage du cordon est connecté électriquement à la masse du générateur de signaux électriques situé dans le boîtier. Le générateur peut être aussi constitué par un générateur du type décrit en référence à la figure 1.

L'instrument 120 auquel aboutit le cordon de liaison comporte un corps allongé 124, essentiellement en matériau isolant formant un élément de préhension et prolongé par l'électrode 121, métallique. Au moins une partie extérieure 122 dudit corps est électriquement isolée de l'électrode 121 mais conductrice de l'électricité et connectée à un premier fil électrique d'alimentation 115 dudit cordon de liaison. Ce premier fil électrique est donc destiné à être relié à l'une des bornes de sortie du générateur électrique situé dans le boîtier. Dans l'exemple, le corps allongé 124, en matériau isolant, comporte un canal central 126 qui aboutit à l'électrode 121 et la partie extérieure 122 du corps qui est conductrice de l'électricité est constituée par une enveloppe métallique tubulaire s'étendant sur une partie notable du corps 124, tout autour d'un manchon en matériau isolant de celui-ci. Intérieurement, une pièce métallique annulaire 128 s'étend entre l'enveloppe métallique et le canal 126 ménagé à l'intérieur du corps et ledit premier fil électrique d'alimentation 115 est connecté à cette pièce qui assure la liaison électrique entre ce fil et ladite enveloppe métallique.

D'autre part, un second fil électrique 116 issu du cordon 118 s'étend à l'intérieur du canal et traverse longitudinalement le corps. Il est connecté à l'électrode 121 et reste bien entendu électriquement isolé de la partie extérieure 122 du corps, conductrice de l'électricité, à savoir l'enveloppe métallique. Le cordon 18 est engagé dans le canal 126 à l'arrière de l'instrument. L'extrémité avant du corps est équipée d'un connecteur 130 auquel ledit second fil électrique 116 est connecté. Ce connecteur est muni d'un embout de raccordement de l'électrode 121. Cette dernière est donc amovible. Dans l'exemple de la figure 6, il s'agit d'une électrode triangulaire permettant un traitement sur des bandes relativement larges de la peau. Elle peut être remplacée par une électrode 121 a en forme de peigne comme représenté à la figure 7 qui permet un traitement selon plusieurs bandes étroites parallèles, simultanément.

Grâce à l'instrument 120 qui vient d'être décrit, il est possible d'appliquer facilement le traitement d'électrothérapie à soi-même. Il suffit de tenir fermement l'instrument par la partie extérieure 122 conductrice de l'électricité (c'est-à-dire par l'enveloppe métallique) pour que le circuit électrique s'établisse naturellement à l'intérieur du corps et qu'un courant électrique de très faible intensité puisse traverser la peau à l'endroit où l'électrode est appliquée sur le corps humain.

## Revendications

1. Appareil de stimulation et de traitement de la peau, comportant des moyens générateurs (11) pour élaborer un signal haute fréquence ayant une fréquence de l'ordre de grandeur de 1 MHz, modulé en amplitude par un signal basse fréquence ayant une fréquence de l'ordre de grandeur de 50 Hz, sous une tension variable de 200V et 1200V, une partie métallique reliée aux moyens générateurs et apte à être tenue à la main par un patient pendant le traitement et une électrode (23) reliée à la sortie desdits moyens générateurs et apte à être déplacée sur une zone de peau à traiter tout en lui appliquant le signal.

2. Appareil selon la revendication 1, **caractérisé en ce que** lesdits moyens générateurs comprennent un hacheur (12) piloté à ladite haute fréquence et recevant en entrée une tension basse fréquence.

3. Appareil selon la revendication 2, **caractérisé en ce qu'**il comporte un transformateur haute fréquence (21) ou transformateur d'impulsions qui est connecté entre ledit hacheur (12) et ladite électrode (23) et qui est adapté à appliquer ledit signal à l'électrode à ladite tension variable de 200V à 1200V.

4. Appareil selon l'une quelconque des revendications précédentes, **caractérisé en ce que** ladite électrode (23a) a la forme d'une fourche dont chaque branche est terminée par une petite excroissance (28), par exemple sensiblement sphérique.

5. Appareil selon l'une quelconque des revendications 1 à 4, **caractérisé en ce que** ladite électrode (23b) a la forme d'une fourche dont chaque branche est terminée par une petite barrette (30) s'étendant transversalement.

6. Appareil selon l'une quelconque des revendications précédentes, **caractérisé en ce qu'**il est complété par un instrument comportant un corps allongé formant élément de préhension, prolongé longitudinalement par ladite électrode reliée aux moyens générateurs et **en ce que** ladite partie métallique apte à être tenue à la pain est formée d'au moins une partie extérieure (122) dudit corps qui est conductrice de l'électricité, électriquement isolée de ladite électrode (121) et connectée à un premier fil électrique d'alimentation (115) destiné à être relié aux dits moyens générateurs.

7. Appareil selon la revendication 6, **caractérisé en ce que** ladite partie extérieure (122) dudit corps comporte une enveloppe métallique.

8. Appareil selon la revendication 7, **caractérisé en ce que** le corps allongé est en matériau isolant et cette enveloppe métallique s'étend tout autour d'un manchon en matériau isolant faisant partie de ce corps allongé.

9. Appareil selon l'une quelconque des revendications 6 à 8, **caractérisé en ce que** l'intérieur dudit corps est traversé par un second fil électrique d'alimentation (116) connecté à ladite électrode (121) et électriquement isolé de ladite partie extérieure dudit corps.

10. Appareil selon la revendication 9, **caractérisé en ce que** lesdits premier et second fils (115, 116) font partie d'un cordon de liaison (118) du type bifilaire blindé.

11. Appareil selon la revendication 10, **caractérisé en ce que** le cordon de liaison est engagé, à l'arrière de l'instrument, dans un canal central du corps (126) dans lequel s'étend le second fil électrique et qui aboutit à l'électrode.

12. Appareil selon l'une quelconque des revendications 6 à 11, **caractérisé en ce que** l'électrode est amovible, une extrémité dudit corps étant équipée d'un connecteur (130) auquel ledit second fil électrique (116) est connecté et ce connecteur étant muni d'un embout de raccordement de cette électrode amovible.

13. Appareil selon la revendication 12, **caractérisé en ce que** ledit instrument est associé à un jeu d'électrodes amovibles (121, 121a) de formes différentes, chacune adaptable audit embout de raccordement.

14. Appareil selon l'une quelconque des revendications 6 à 13, **caractérisé en ce que** l'électrode a une forme triangulaire.

15. Appareil selon l'une quelconque des revendications 6 à 13, **caractérisé en ce que** l'électrode a la forme d'un peigne.

16. Utilisation d'un appareil selon l'une quelconque des revendications 1 à 15, en appliquant le signal sous une tension variable de 200V à 1200V, via un transformateur d'impulsions, pour le traitement des rides en vue de les diminuer.

17. Utilisation d'un appareil selon l'une quelconque des revendications 1 à 16, en appliquant le signal sous une tension variable de 200V à 1200V, via un transformateur d'impulsions, pour agir sur le métabolisme des cheveux.

## Claims

1. Device for stimulation and treatment of the skin, including generator means (11) for producing a high-frequency signal having a frequency of the order of magnitude of 1 MHz, amplitude-modulated by a low-frequency signal having a frequency of the order of magnitude of 50 Hz, at a voltage variable from 200 V to 1200 V, a metal part connected to the generator means and adapted to be held in the hand by a patient during the treatment, and an electrode (23) connected to the output of said generator means and adapted to be moved over an area of skin to be treated whilst applying the signal to it.

2. Device according to claim 1, **characterised in that** said generator means comprise a chopper (12) driven at said high frequency and receiving at its input a low-frequency voltage.

3. Device according to claim 2, **characterised in that** it includes a high-frequency transformer (21) or pulse transformer that is connected between said chopper (12) and said electrode (23) and that is adapted to apply said signal to the electrode at said voltage variable from 200 V to 1200 V.

4. Device according to any of the preceding claims, **characterised in that** said electrode (23a) has the shape of a fork each branch of which is terminated by a small, for example substantially spherical, excrescence (28).

5. Device according to any of claims 1 to 4, **characterised in that** said electrode (23b) has the shape of a fork each branch of which is terminated by a small strip (30) extending transversely.

6. Device according to any of the preceding claims, **characterised in that** it is complemented by an instrument including an elongate body forming a holding member, extended longitudinally by said electrode connected to the generator means and **in that** said metal part adapted to be held in the hand is formed of at least one exterior part (122) of said body that is electrically conductive, electrically insulated from said electrode (121) and connected to a first power supply electrical wire (115) adapted to be connected to said generator means.

7. Device according to claim 6, **characterised in that** said exterior part (122) of said body includes a metal envelope.

8. Device according to claim 7, **characterised in that** the elongate body is in insulative material and this metal envelope extends all around a sleeve in insulative material forming part of this elongate body.

9. Device according to any of claims 6 to 8, **characterised in that** the interior of said body has passing through it a second power supply electrical wire (116) connected to said electrode (121) and electrically insulated from said exterior part of said body.

10. Device according to claim 9, **characterised in that** said first and second wires (115, 116) form part of a screened two-wire connecting cord (118).

11. Device according to claim 10, **characterised in that** the connecting cord is engaged, at the rear of the instrument, in a central passage of the body (126) in which the second electrical wire extends and that ends at the electrode.

12. Device according to any of claims 6 to 11, **characterised in that** the electrode is removable, one end of said body being equipped with a connector (130) to which said second electrical wire (116) is connected and this connector being provided with a terminal for connecting this removable electrode.

13. Device according to claim 12, **characterised in that** said instrument is associated with a set of removable electrodes (121, 121a) of different shapes, each adapted to be fitted to said connection terminal.

14. Device according to any of claims 6 to 13, **characterised in that** the electrode has a triangular shape.

15. Device according to any of claims 6 to 13, **characterised in that** the electrode has the shape of a comb.

16. Use of a device according to any of claims 1 to 15, by applying the signal at a voltage variable from 200 V to 1200 V, via a pulse transformer, for the treatment of wrinkles with a view to reducing them.

17. Use of a device according to any of claims 1 to 16 by applying the signal at a voltage variable from 200 V to 1200 V via a pulse transformer to operate on the metabolism of the hair.

## Patentansprüche

1. Gerät zur Stimulierung und Behandlung der Haut, umfassend Generatormittel (11) zur Erzeugung eines Hochfrequenzsignals mit einer Frequenz von der Größenordnung von 1 MHz, das durch ein Niederfrequenzsignal mit einer Frequenz von der Größenordnung von 50 Hz amplitudenmoduliert ist, unter einer von 200V bis 1200V veränderlichen Spannung, einen metallischen Teil, der mit den Generatormitteln verbunden ist und von einem Patienten während der Behandlung in der Hand gehalten werden kann, und eine Elektrode (23), die mit dem Ausgang der Generatormittel verbunden ist und auf einer zu behandelnden Hautzone bewegt werden kann, indem das Signal an sie angelegt wird.

2. Gerät nach Anspruch 1, **dadurch gekennzeichnet, dass** die Generatormittel einen Zerhacker (12) umfassen, der mit der Hochfrequenz gesteuert wird und am Eingang eine Niederfrequenzspannung erhält.

3. Gerät nach Anspruch 2, **dadurch gekennzeichnet, dass** es einen Hochfrequenztransformator (21) oder Impulstransformator umfasst, der zwischen den Zerhacker (12) und die Elektrode (23) geschaltet ist und der dafür ausgelegt ist, das Signal an die Elektrode mit der von 200V bis 1200V veränderlichen Spannung anzulegen.

4. Gerät nach einem der vorhergehenden Ansprüche, **dadurch gekennzeichnet, dass** die Elektrode (23a) die Form einer Gabel aufweist, von der jeder Schenkel mit einem kleinen, beispielsweise im Wesentlichen kugelförmigen Auswuchs (28) endet.

5. Gerät nach einem der Ansprüche 1 bis 4, **dadurch gekennzeichnet, dass** die Elektrode (23b) die Form einer Gabel aufweist, von der jeder Schenkel mit einem kleinen, sich in Querrichtung erstreckenden Stab (30) endet.

6. Gerät nach einem der vorhergehenden Ansprüche, **dadurch gekennzeichnet, dass** es durch ein Instrument vervollständigt ist, das einen langgestreckten, ein Greifelement bildenden Körper umfasst, der in Längsrichtung durch die mit den Generatormitteln verbundene Elektrode verlängert ist, und dass der metallische Teil, der in der Hand gehalten werden kann, von mindestens einem äußeren Teil (122) des Körpers gebildet ist, der elektrisch leitend ist, von der Elektrode (121) elektrisch isoliert ist und an einen ersten elektrischen Versorgungsdraht (115) angeschlossen ist, der dazu bestimmt ist, mit den Generatormitteln verbunden zu werden.

7. Gerät nach Anspruch 6, **dadurch gekennzeichnet, dass** der äußere Teil (122) des Körpers eine metallische Hülle umfasst.

8. Gerät nach Anspruch 7, **dadurch gekennzeichnet, dass** der langgestreckte Körper aus einem isolierenden Werkstoff besteht und die metallische Hülle sich ganz um eine Buchse aus isolierendem Werkstoff herum erstreckt, die Teil dieses langgestreckten Körpers bildet.

9. Gerät nach einem der Ansprüche 6 bis 8, **dadurch gekennzeichnet, dass** das Innere des Körpers von einem zweiten elektrischen Versorgungsdraht (116) durchquert wird, der an die Elektrode (121) angeschlossen ist und von dem äußeren Teil des Körpers elektrisch isoliert ist.

10. Gerät nach Anspruch 9, **dadurch gekennzeichnet, dass** der erste und der zweite Draht (115, 116) Teil eines Verbindungskabels (118) vom Typ abgeschirmte Zweidrahtleitung bilden.

11. Gerät nach Anspruch 10, **dadurch gekennzeichnet, dass** das Verbindungskabel im hinteren Teil des Instruments in einen zentralen Kanal des Körpers (126) eingeführt ist, in dem sich der zweite elektrische Draht erstreckt und der zur Elektrode führt.

12. Gerät nach einem der Ansprüche 6 bis 11, **dadurch gekennzeichnet, dass** die Elektrode abnehmbar ist, wobei ein Ende des Körpers mit einem Verbinder (130) ausgerüstet ist, an den der zweite elektrische Draht (116) angeschlossen ist, und dieser Verbinder mit einem Endstück zum Anschluss der abnehmbaren Elektrode versehen ist.

13. Gerät nach Anspruch 12, **dadurch gekennzeichnet, dass** dem Instrument ein Satz von abnehmbaren Elektroden (121, 121a) von verschiedenen Formen zugeordnet ist, deren jede an das Anschlussendstück anpassbar ist.

14. Gerät nach einem der Ansprüche 6 bis 13, **dadurch gekennzeichnet, dass** die Elektrode eine dreieckige Form aufweist.

15. Gerät nach einem der Ansprüche 6 bis 13, **dadurch gekennzeichnet, dass** die Elektrode die Form eines Kamms aufweist.

16. Verwendung eines Geräts nach einem der Ansprüche 1 bis 15, indem das Signal unter einer von 200V bis 1200V veränderlichen Spannung über einen Impulstransformator für die Behandlung von Falten angelegt wird, um sie zu verringern.

17. Verwendung eines Geräts nach einem der Ansprüche 1 bis 16, indem das Signal unter einer von 200V bis 1200V veränderlichen Spannung über einen Impulstransformator angelegt wird, um auf den Stoffwechsel der Haare einzuwirken.
